# EUROPEAN PATENT APPLICATION

(11) **EP 4 707 785 A1**
(43) Date of publication of application: **11.03.2026**
(21) Application number: 24797418.1
(22) Date of filing: 25.04.2024
(51) Int. Cl.: G01N 21/84, G01N 1/28, G01N 33/22, G06N 3/08, G06N 3/04, G01N 21/17, G01N 21/88

(54) **MATERIAL ANALYSIS METHOD**

(30) Priority: 28.04.2023 KR 20230056500
(71) Applicant: Hyundai Steel Company, Incheon 22525 (KR)
(72) Inventor: PARK, Tae Chang, Incheon 22525 (KR); KIM, Nam Uk, Incheon 22525 (KR); KIM, Byong Chul, Incheon 22525 (KR)
(74) Representative: Viering, Jentschura & Partner mbB Patent- und Rechtsanwälte
(86) International application number: PCT/KR2024/005586
(87) International publication number: WO 2024/225763

(57) **Abstract**

The present invention relates to a material analysis method which may comprise the steps of: (a) preparing a specimen for structure analysis; (b) training an artificial intelligence model with a training image labelled with a structure aspect of an arbitrary specimen; (c) analyzing a plurality of analysis images not labelled with a structure aspect of a specimen using the trained artificial intelligence model, and removing analysis images classified into preset noise structure aspects; and (d) analyzing, by using the trained artificial intelligence model, the analysis images from which the analysis images classified into the noise structure aspects have been removed, and classifying the analysis images into at least one target structure aspect.

## Description

### TECHNICAL FIELD

The present invention relates to a material analysis method and, more specifically, to a method for analyzing the microstructure of a material using artificial intelligence.

### BACKGROUND ART

In the coke-making process of a steelworks, coal is subjected to pyrolysis at a high temperature of 1,000 to 1,300°C to produce coke suitable for use in the blast furnace. The coal used as a feedstock for coke is a carbonaceous mineral consisting of a heterogeneous mixture of microstructural constituents known as macerals.

Macerals, which are organic components distinguished by their size and morphology mainly using a microscope, serve as the minimum classification units in the microstructure analysis of coal. According to the KS standard (KS E ISO 7404-3), the classification of macerals is fundamentally dependent on point-counting visual inspection by the operator. This method requires the operator to manually count at least 500 points, resulting in significant variability among different operators and being highly time-consuming.

### DETAILED DESCRIPTION OF THE INVENTION

### TECHNICAL PROBLEM

Accordingly, the present invention is directed to solving the above, as well as various other, challenges by providing a material analysis method that enables the identification and quantification of coal macerals to be automated using artificial intelligence (AI) models based on deep learning, thereby replacing conventional maceral analysis that relied on visual inspection by a limited number of skilled operators. The invention aims to provide a technique whereby even non-expert operators can easily and rapidly classify coal macerals and determine their proportion. It should be understood, however, that this object is presented by way of example only and that the scope of the present invention is not limited thereby.

### TECHNICAL SOLUTION

According to one embodiment of the present invention, a material analysis method is provided. The material analysis method may comprise (a) preparing a specimen for microstructure analysis; (b) capturing a plurality of analytical images by photographing an inspection area defined on a surface of the specimen; (c) analyzing the plurality of analytical images using a trained first artificial intelligence (AI) model to remove images classified as exhibiting a predetermined noise microstructure pattern; and (d) analyzing the remaining analytical images, from which the images classified as exhibiting the noise microstructure pattern have been removed, using a trained second artificial intelligence (AI) model to classify the remaining images into at least one target microstructure pattern.

In one embodiment, step (a) may include (a-1) crushing the material into particles having a predetermined size, and (a-2) forming the specimen by mixing the particles with a binder.

The material may be coal, and the binder may be epoxy.

The first artificial intelligence (AI) model may be a noise removal Al model that is trained to learn and classify noise microstructure patterns to distinguish noise present in the specimen. The second artificial intelligence (AI) model may be a microstructure classification Al model that is trained to learn and classify target microstructure patterns to distinguish microstructure patterns present in the specimen.

The noise removal Al model may be implemented using a deep learning network based on a residual neural network or MobileNet.

The microstructure classification Al model may be implemented using a deep learning network based on Inception network.

According to one embodiment of the present invention, the material analysis method may further comprise, prior to step (c), step (b'), in which labeled training images of microstructure patterns of arbitrary specimens are used to train the artificial intelligence (AI) model.

According to one embodiment of the present invention, step (b') may include (b'-1) training the noise removal artificial intelligence (AI) model using noise training images and (b'-2) training the microstructure classification artificial intelligence (AI) model using target microstructure training images.

According to one embodiment of the present invention, in step (c), the noise microstructure pattern may refer to an image in which the microstructure pattern of the specimen is classified as a binder by the first artificial intelligence (AI) model.

According to one embodiment of the present invention, in step (d), the target microstructure pattern may be an image in which the microstructure pattern of the specimen is classified, by the second artificial intelligence (AI) model, as at least one selected from the group consisting of vitrinite, exinite, fusinite, semi-fusinite, mineral, and combinations thereof.

According to one embodiment of the present invention, the method may further comprise, after step (d), step (e) of calculating a proportion of each microstructure pattern classified in the specimen.

### ADVANTAGEOUS EFFECTS

According to an embodiment of the present invention as set forth above, by defining a hexagonal inspection area, the characteristics of the entire circular cross-section can be sufficiently reflected, and efficient analysis time can be ensured. Moreover, by utilizing artificial intelligence, it is possible to easily and rapidly distinguish maceral microstructures that would otherwise be difficult for an ordinary operator to differentiate.

Furthermore, by independently using an artificial intelligence (AI) model for classifying epoxy images and another Al model for classifying maceral microstructures, maceral microstructures can be classified with even greater precision. Of course, the scope of the present invention is not limited by these effects.

### DESCRIPTION OF THE DRAWINGS

Figure 1 is a flowchart illustrating, in sequence, the steps of the material analysis method according to an embodiment of the present invention.
Figure 2 is a flowchart illustrating in greater detail each step of the material analysis method according to an embodiment of the present invention.
Figure 3 is a schematic diagram illustrating the structure of the noise removal artificial intelligence (AI) model according to an embodiment of the present invention.
Figure 4 is a schematic diagram illustrating the structure of the microstructure classification artificial intelligence (AI) model according to an embodiment of the present invention.
Figure 5 is a table and image illustrating the confusion matrix and the structure of the macerals according to an embodiment of the present invention.
Figure 6 is a table showing the accuracy of the noise removal artificial intelligence (AI) models with respect to initial learning rate.
Figure 7 is a table showing the accuracy of the microstructure classification artificial intelligence (AI) models with respect to initial learning rate.
Figure 8 is a conceptual diagram schematically illustrating the material analysis apparatus according to an embodiment of the present invention.
FIG. 9 is an illustrative image showing a hexagonal inspection region according to one embodiment of the present invention.

### MODE OF THE INVENTION

Below, preferred embodiments of the present invention will be described in detail with reference to the accompanying drawings.

These embodiments are provided to more fully explain the invention to those skilled in the art and may be modified in various forms without departing from the scope of the present invention. The scope of the invention is not limited to the following embodiments. Rather, these embodiments are presented to enhance the completeness and understanding of the present disclosure, and to fully convey the inventive concept to those skilled in the art. Furthermore, the thicknesses and sizes of layers shown in the drawings are exaggerated for the sake of clarity and convenience of explanation.

The following embodiments of the present invention will be described with reference to the drawings that schematically illustrate ideal embodiments of the invention. It should be noted that modifications to the illustrated shapes may be anticipated due to, for example, manufacturing techniques and/or tolerances. Therefore, the embodiments of the inventive concept should not be construed as being limited to the specific shapes of regions depicted in the specification, and should be understood to include variations in shape resulting from manufacturing processes, for example.

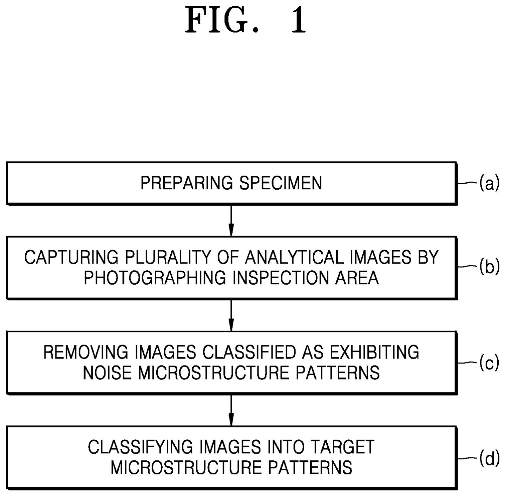

Figure 1 is a flowchart illustrating, in sequence, each step of a material analysis method according to one embodiment of the present invention.

As shown in Figure 1, the material analysis method of the present embodiment may comprise (a) preparing a specimen for microstructure analysis; (b) capturing a plurality of analytical images by photographing an inspection area defined on a surface of the specimen; (c) analyzing the plurality of analytical images using a trained first artificial intelligence (AI) model to remove images classified as exhibiting a predetermined noise microstructure pattern; and (d) analyzing the remaining analytical images, from which the images classified as exhibiting the noise microstructure pattern have been removed, using a trained second artificial intelligence (AI) model to classify the remaining images into at least one target microstructure pattern.

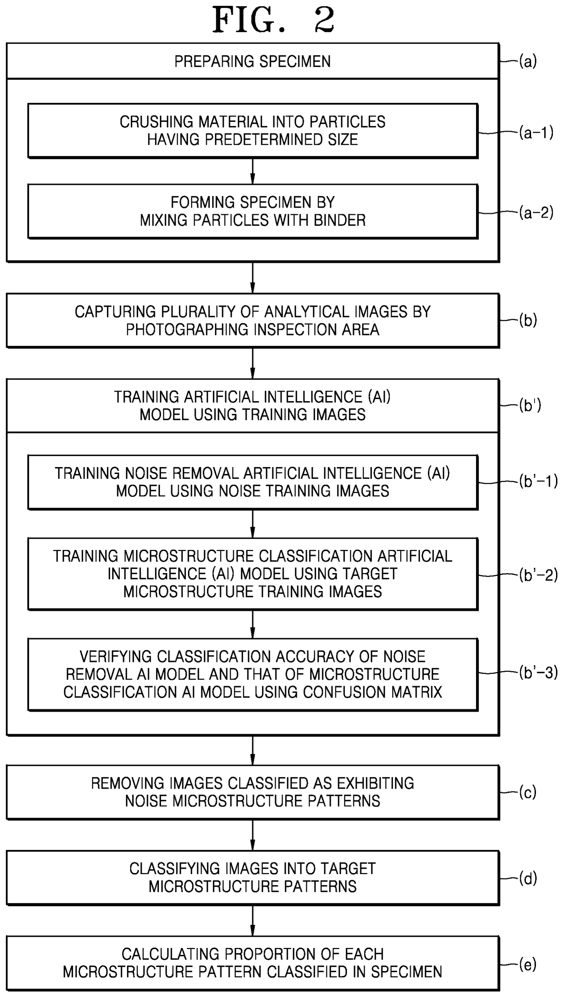

More specifically, as illustrated in FIG. 2, step (a) of preparing a specimen for microstructure analysis may comprise (a-1) crushing the material into particles having a predetermined size, and (a-2) fabricating the specimen by mixing the particles with a binder.

In this embodiment, the material is coal and the binder is epoxy, so the process may involve crushing coal into particles of a predetermined size, mixing the particles with the binder, and then performing pressure molding to fabricate a specimen having an overall cylindrical shape.

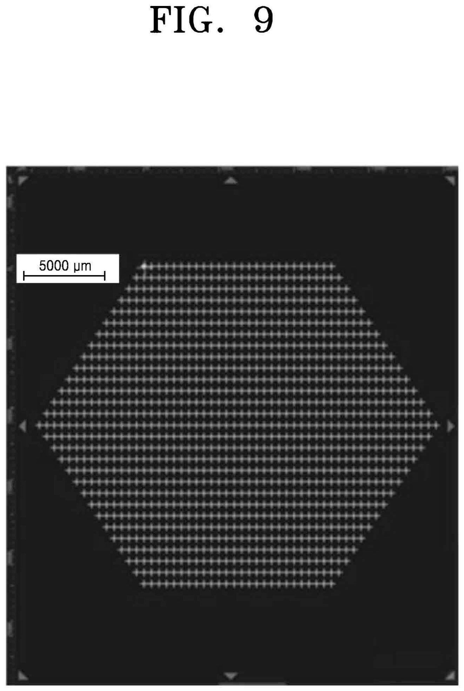

In subsequent step (b), which involves capturing a plurality of analytical images by photographing an inspection area defined on a surface of the specimen, the inspection area may be an area in which a plurality of inspection points are arranged so as to form an overall hexagonal shape. As subsequently illustrated in Figure 9, the inspection area may be defined such that the plurality of inspection points together form a hexagonal shape.

In addition, the artificial intelligence (AI) models applied in the material analysis method of the present invention may include a noise removal Al model, which is trained to learn and classify noise microstructure patterns so as to identify and distinguish noise present in the specimen, and a microstructure classification Al model, which is trained to learn and classify target microstructure patterns so as to identify and distinguish microstructure patterns present in the specimen. In this context, the noise microstructure pattern may refer to a binder, that is, an epoxy image, while the target microstructure pattern may refer to an image of coal maceral microstructure.

Specifically, in step (c), which involves analyzing the plurality of analytical images and removing images classified as exhibiting a predetermined noise microstructure pattern, the first artificial intelligence (AI) model may be a noise removal Al model. In step (d), which involves analyzing the remaining analytical images-those from which images classified as exhibiting the noise microstructure pattern have been removed-and classifying the remaining images into at least one target microstructure pattern, the second artificial intelligence (AI) model may be a microstructure classification Al model.

More concretely, the noise removal Al model may be trained to identify and classify as noise microstructure patterns the images in which the microstructure pattern of the specimen is classified as a binder (i.e., epoxy). The microstructure classification Al model may be trained to identify and classify as target microstructure patterns the images in which the microstructure pattern of the specimen is classified as at least one selected from the group consisting of vitrinite, exinite, fusinite, semi-fusinite, mineral, and combinations thereof.

In addition, as illustrated in Figure 2, the material analysis method according to an embodiment of the present invention may further include, prior to step (c) for removing images classified as exhibiting a noise microstructure pattern, step (b'), in which labeled training images of microstructure patterns of arbitrary specimens are used to train the artificial intelligence (AI) model.

The steps (a) through (d) depicted in Figure 2 are not necessarily performed in the order presented. For example, after training the Al model using arbitrary specimens in step (b'), a specimen for microstructure analysis may be prepared in step (a).

More specifically, step (b'), in which training images are provided to the artificial intelligence (AI) models, may include training the noise removal Al model using noise training images and training the microstructure classification Al model using target microstructure training images.

In this case, in step (b'-1) for training the noise removal Al model using noise training images, the noise training images may be labeled images in which the microstructure pattern of the specimen is classified as a binder.

In addition, in step (b'-2) for training the microstructure classification Al model using target microstructure training images, the target microstructure training images may be labeled images in which the microstructure pattern of the specimen is classified as at least one selected from the group consisting of vitrinite, exinite, fusinite, semi-fusinite, mineral, and combinations thereof.

Hereinafter, the (c) step through (d) step of the material analysis method according to an embodiment of the present invention will be described in detail.

According to one embodiment of the present invention, in step (c), by using the noise removal artificial intelligence (AI) model to preliminarily remove images classified as exhibiting a predetermined noise microstructure pattern among the plurality of analytical images of the specimen, it is possible to reduce the number of analytical images to be classified in step (d), and the remaining analytical images can be efficiently classified into at least one target microstructure pattern using the microstructure classification Al model.

In step (c) of the material analysis method according to an embodiment of the present invention, the noise removal Al model may be an artificial intelligence (AI) model using a deep learning network based on a residual neural network (ResNet) or MobileNet.

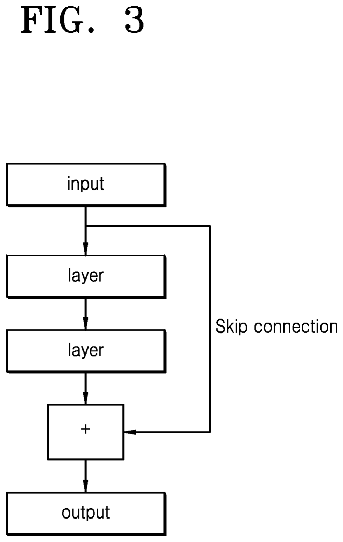

As illustrated in Figure 3, the residual neural network (ResNet) artificial intelligence (AI) model introduces a skip connection, which enables the model to learn the differences between preceding and subsequent layers. By calculating and learning the changes across layers based on the skip connection, the model effectively addresses the issue of information loss that arises as the neural network depth increases.

According to an embodiment of the present invention, the skip connection structure of the ResNet ensures that features of epoxy images-which display a relatively simple form compared to the complex coal maceral microstructures-can be effectively extracted and propagated to subsequent layers. Even as the network depth increases, the extracted features do not become blurred, thereby enabling high-precision classification of epoxy images.

Although not shown in the drawings, MobileNet is designed to analyze images in real time on mobile devices. Due to its small model size and low computational cost, MobileNet provides high classification accuracy and enables fast and accurate classification of only epoxy images.

In step (d) of the material analysis method according to one embodiment of the present invention, the microstructure classification artificial intelligence (AI) model may be an Al model utilizing a deep learning network based on Inception. More specifically, the Al model may employ a deep learning network based on Inception V3.

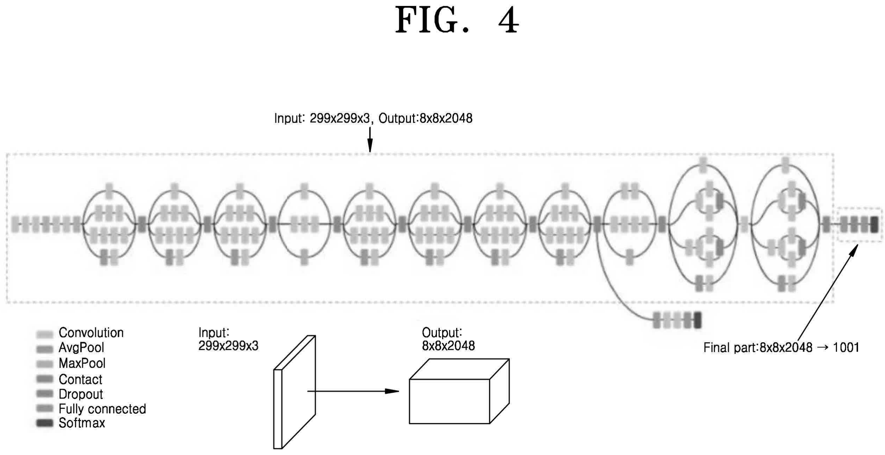

As illustrated in Figure 4, the Inception V3 Al model may include an Inception module composed of a combination of convolution layer, average pooling layer, max pooling layer, concatenation (Concat), Dropout, and Softmax. Each Inception module may be structured to compute divided convolution layers of various sizes, such as 1x1, 3x3, and 5x5, thereby reducing computational load while extracting features from input images through multiple stages.

According to an embodiment of the present invention, the use of divided convolution layers, which is a distinguishing feature of the Inception architecture, allows the artificial intelligence (AI) model to find a greater variety of features compared to an Al model having a single layer.

As a result, it is possible to effectively classify images of coal maceral microstructures, which contain various microstructure patterns. Accordingly, when analyzing images of the specimen using a single Al model, the precision for distinguishing epoxy did not satisfy the required level and remained in the 40% range. However, by independently employing the noise removal Al model for classifying epoxy images and the microstructure classification Al model for classifying maceral microstructure images, the classification of epoxy images was achieved with a precision in the 99% range, and the classification of maceral microstructure images was achieved with a precision in the 94% range.

In addition, step (b') of the material analysis method according to an embodiment of the present invention may further include step (b'-3), in which the classification accuracy of the trained artificial intelligence (AI) models is verified using a confusion matrix. More specifically, step (b'-3) may include verifying the classification accuracy of the noise removal Al model and that of the microstructure classification Al model using the confusion matrix.

A confusion matrix may be used to visualize the results of predictions made by an artificial intelligence (AI) model in relation to classification data, in the form of a matrix. For example, a confusion matrix may be provided as a 2×2 matrix, in which the combinations of true and false values for both predicted values and actual values are arranged. In this case, a true positive refers to a case in which the actual positive value matches the predicted positive value, a false positive refers to a case in which a negative value is incorrectly predicted as positive, a true negative refers to a case in which the actual negative value matches the predicted negative value, and a false negative refers to a case in which a positive value is incorrectly predicted as negative.

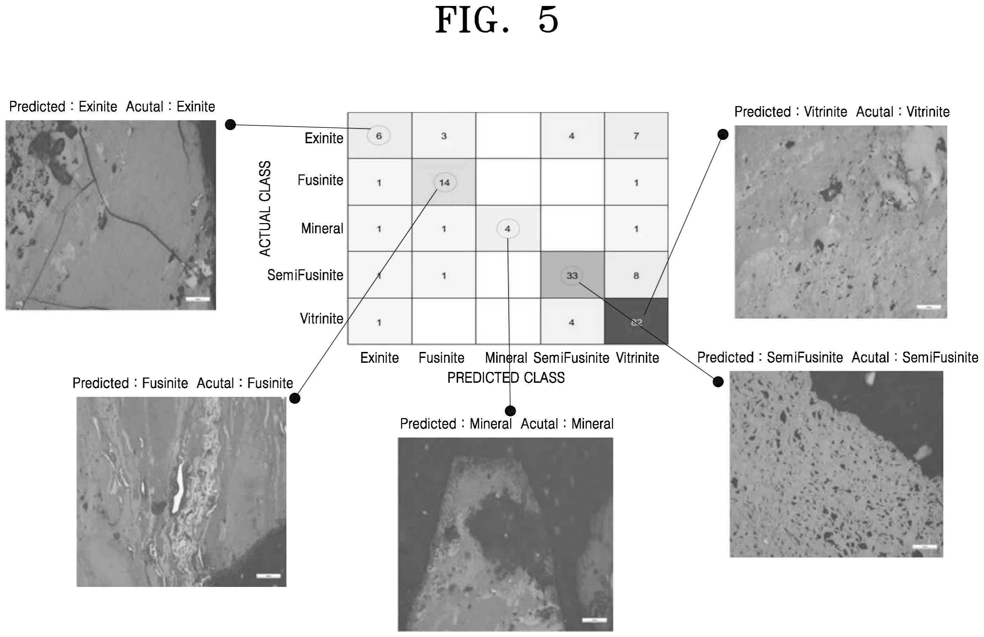

In an embodiment of the present invention, the confusion matrix is extended to a 5x5 table, as shown in Figure 5, in order to classify the coal microstructure constituents (maceral) into five categories. In this case, true positive values, where the predicted positive matches the actual positive, are located along the diagonal from the top left to the bottom right of the matrix, while the other values may be combinations of false positives or false negatives.

The composition of the macerals in coal can be classified, as shown in Figure 5, into vitrinite, exinite, fusinite, semi-fusinite, and mineral.

More specifically, vitrinite accounts for most of the caking constituents of coal and is derived from the woody tissues of plants, exhibiting a smooth and homogeneous microstructure surface. Exinite is derived from plant leaves, small branches, or barks used for adjusting caking properties, and is characterized by high volatile matter and tar content, generally exhibiting a dark brown, serrated microstructure. Inertinite represents an inert component that does not soften or melt upon heating and may have a three-dimensional microstructure. The major types of inertinite include fusinite and semi-fusinite. Mineral refers to a small portion of inorganic minerals contained in coal, typically exhibiting a dark brown microstructure.

In conventional maceral analysis by visual inspection, it is very difficult for an ordinary operator to distinguish macerals, and the results have depended heavily on the expertise of highly skilled operators. However, by applying the material analysis method of the present embodiment, even ordinary operators can easily and quickly distinguish maceral microstructures that are difficult to differentiate manually. For example, it becomes possible to readily distinguish semi-fusinite, which exhibits characteristics intermediate between vitrinite and fusinite, and to distinguish between exinite and mineral, both of which tend to have a dark brown appearance.

In step (d), which involves classifying analytical images into target microstructure patterns, images of epoxy are removed as noise microstructure patterns in step (c), and the remaining analytical images are then examined and classified into at least one of the aforementioned microstructure patterns, including vitrinite, exinite, fusinite, semi-fusinite, mineral, and combinations thereof.

Furthermore, the material analysis method according to an embodiment of the present invention may further include, after step (d), step (e) of calculating the proportion of each classified microstructure pattern in the specimen. For example, the maceral microstructure of the coal specimen to be analyzed can be quantified as the proportion (%) of vitrinite, exinite, fusinite, semi-fusinite, and mineral with respect to the total volume.

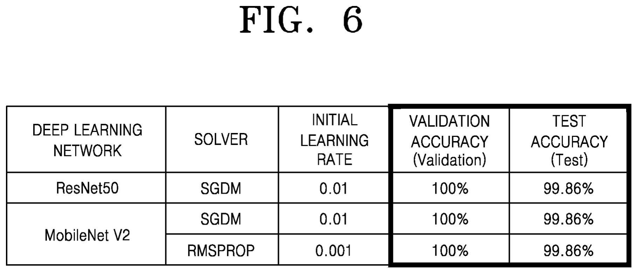

Figure 6 is a table comparing the validation accuracy and test accuracy of the deep learning networks, ResNet-50 and MobileNet V2, which serve as artificial intelligence (AI) models in the material analysis method according to an embodiment of the present invention.

When the two types of deep learning networks described above were applied as the noise removal artificial intelligence (AI) model in the material analysis method according to an embodiment of the present invention to perform step (c) of detecting noise, specifically epoxy images, both networks demonstrated exceptionally high accuracy, with a validation accuracy of 100% and a test accuracy of 99.86%, respectively, in distinguishing epoxy images.

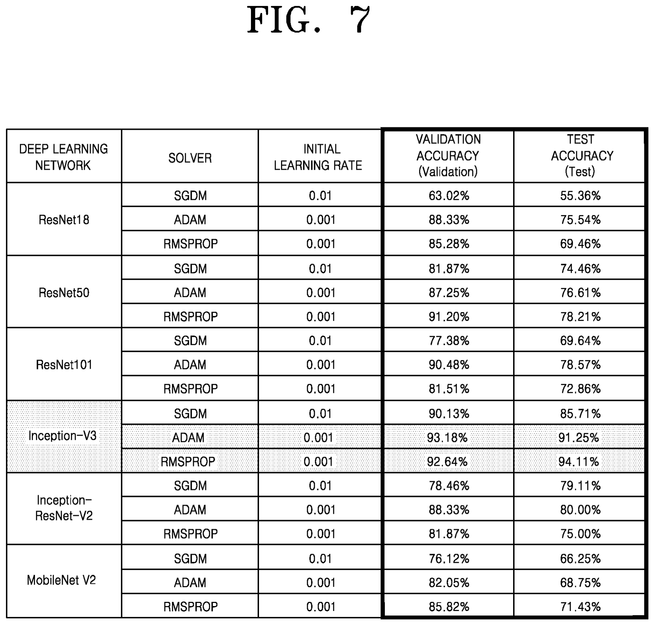

Figure 7 is a table comparing the validation accuracy and test accuracy of the Inception V3 deep learning network and other types of deep learning networks, each serving as the artificial intelligence (AI) model of the material analysis method according to an embodiment of the present invention.

As shown in Figure 7, for each of the deep learning networks-ResNet, Inception-ResNet, MobileNet, and Inception V3 as applied in the present invention-the validation accuracy and test accuracy were measured after applying three different solver parameters with initial learning rates of 0.01 or 0.001. The results confirmed that Inception V3 exhibited the highest validation accuracies (93.18% and 92.64%) and the highest test accuracies (91.25% and 94.11%) when the initial learning rate was set to 0.001.

Through repeated experimentation, the present applicant confirmed that, when applying a combination of the residual neural network (ResNet) Al model and the Inception V3 Al model, or a combination of the MobileNet Al model and the Inception V3 Al model, with the ADAM or RMSPROP solver and an initial learning rate of 0.001, it was possible to achieve the highest accuracy in classifying coal maceral microstructures.

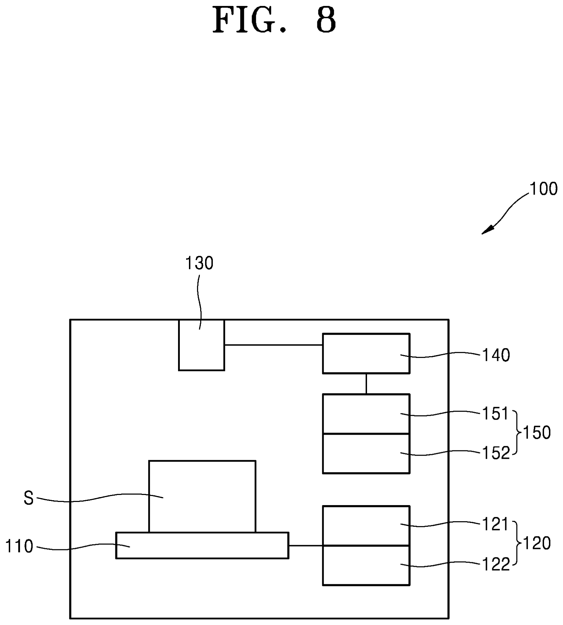

Hereinafter, embodiments of a material analysis apparatus 100 according to the present invention will be described with reference to Figures 8 through 9.

Figure 8 is a conceptual diagram schematically illustrating the material analysis apparatus 100 according to an embodiment of the present invention. As shown in Figure 8, the material analysis apparatus 100 may include a stage 110, a stage drive apparatus 120, an image capturing apparatus 130, and a microstructure classification apparatus 140.

More specifically, the stage drive apparatus 120 may include a stage longitudinal drive apparatus 121 configured to move the stage 110 in the forward and backward directions and a stage lateral drive apparatus 122 configured to move the stage 110 in the left and right directions.

The image capturing apparatus 130, as illustrated in Figure 9, is configured to capture an image of the specimen by defining an inspection area on one surface of the specimen. In this case, the inspection area may be an area in which multiple inspection points are arranged so as to form an overall hexagonal shape. As shown in Figure 9, the inspection area may be defined in such a way that the plurality of inspection points together creates a hexagonal shape.

Accordingly, the stage drive apparatus 120 can move the stage 110 so that the image capturing apparatus 130 is positioned at the inspection point located at any one vertex of the hexagon, and then can move the stage 110 forward or backward, or left or right, so that the image capturing apparatus 130 is positioned at an inspection point at another adjacent vertex of the hexagon.

In conventional approaches, the inspection area was generally defined so that the inspection points collectively formed a rectangular shape. However, in the present invention, by configuring the inspection area to form the aforementioned hexagonal shape, it becomes possible to more adequately represent the characteristics of an entire circular cross-section. When the inspection area is set to have a polygonal shape with more than six sides, the excessive number of inspection points required can lead to increased inspection time, making analysis less efficient. Thus, defining the inspection area as a hexagon enables the most efficient analysis.

Furthermore, the material analysis apparatus 100 according to an embodiment of the present invention may include a deep learning unit 150. The deep learning unit 150 may comprise a noise removal deep learning unit 151, which is configured to learn and classify noise microstructure patterns to distinguish noise present in the specimen, and a microstructure classification deep learning unit 152, which is configured to learn and classify target microstructure patterns to distinguish microstructure patterns present in the specimen.

Accordingly, the analytical images of the inspection points of the specimen, which are captured by the image capturing apparatus 130, can be classified into vitrinite, exinite, fusinite, semi-fusinite, and mineral by using an artificial intelligence (AI) model that has been trained through deep learning.

Although the present invention has been described with reference to the embodiments shown in the drawings, these embodiments are provided only as examples. Those skilled in the art will appreciate that various modifications and equivalent other embodiments may be made based on the present disclosure. Therefore, the true scope of technical protection for the present invention should be defined by the appended claims.

## Claims

1. A method for analyzing material comprising:
(a) preparing a specimen for microstructure analysis;
(b) capturing a plurality of analytical images by photographing an inspection area defined on a surface of the specimen;
(c) analyzing the plurality of analytical images using a trained first artificial intelligence (AI) model to remove images classified as exhibiting a predetermined noise microstructure pattern; and
(d) analyzing remaining analytical images, from which the images classified as exhibiting the noise microstructure pattern have been removed, using a trained second artificial intelligence (AI) model to classify the remaining images into at least one target microstructure pattern.

2. The method of claim 1, wherein step (a) comprises:
(a-1) crushing the material into particles having a predetermined size; and
(a-2) forming the specimen by mixing the particles with a binder.

3. The method of claim 2, wherein the material is coal, and the binder is an epoxy.

4. The method of claim 1, wherein the first artificial intelligence (AI) model is a noise removal Al model trained to classify noise microstructure patterns to identify noise present in the specimen; and the second artificial intelligence (AI) model is a microstructure classification Al model trained to classify target microstructure patterns to identify microstructure patterns present in the specimen.

5. The method of claim 4, wherein the noise removal artificial intelligence (AI) model uses a deep learning network based on a residual neural network (ResNet) or MobileNet.

6. The method of claim 4, wherein the microstructure classification Al model is based on a deep learning network using an Inception network.

7. The material analysis method according to claim 1, further comprising, prior to step (c), step (b') of training the artificial intelligence (AI) model using training images in which the microstructure patterns of arbitrary specimens are labeled.

8. The method of claim 7, wherein step (b') comprises: (b'-1) training the noise removal artificial intelligence (AI) model using noise training images; and (b'-2) training the microstructure classification artificial intelligence (AI) model using target microstructure training images.

9. The method of claim 1, wherein, in step (c), the noise microstructure pattern is an image in which the microstructure pattern of the specimen is classified as binder by the first artificial intelligence (AI) model.

10. The method of claim 1, wherein, in step (d), the target microstructure pattern comprises images in which a microstructure pattern of the specimen is classified, by the second artificial intelligence (AI) model, as at least one selected from the group consisting of vitrinite, exinite, fusinite, semi-fusinite, mineral, and combinations thereof.

11. The method of claim 1, further comprising, after step (d), (e) calculating a proportion of each microstructure pattern classified in the specimen.
